# EUROPÄISCHE PATENTSCHRIFT

(11) **EP 3 079 803 B1**
(45) Veröffentlichungstag und Bekanntmachung des Hinweises auf die Patenterteilung: **24.04.2019**
(21) Anmeldenummer: 14796145.2
(22) Anmeldetag: 12.11.2014
(51) Int. Cl.: A01C 1/08, A61L 2/26, G21K 5/00, H01J 33/00

(54) **VORRICHTUNG ZUM BEAUFSCHLAGEN VON SCHÜTTGUT MIT BESCHLEUNIGTEN ELEKTRONEN**
APPARATUS FOR SUBJECTING BULK MATERIAL TO THE ACTION OF ACCELERATED ELECTRONS
DISPOSITIF D'APPLICATION D'ÉLECTRONS ACCÉLÉRÉS À UN PRODUIT EN VRAC

(30) Priorität: 09.12.2013 DE 102013113688
(43) Veröffentlichungstag der Anmeldung: 19.10.2016
(73) Patentinhaber: Fraunhofer-Gesellschaft zur Förderung der angewandten Forschung e.V., 80686 München (DE)
(72) Erfinder: RÖGNER, Frank-Holm, 01277 Dresden (DE); WEIDAUER, André, 01259 Dresden (DE); MATTAUSCH, Gösta, 01454 Ullersdorf (DE); KUBUSCH, Jörg, 01279 Dresden (DE)
(86) Internationale Anmeldenummer: PCT/EP2014/074378
(87) Internationale Veröffentlichungsnummer: WO 2015/086246

(56) Entgegenhaltungen:
- WO-A1-98/43274
- DE-A1- 19 942 142
- DE-A1-102009 034 646
- DE-C1- 4 434 767

## Beschreibung

Die Erfindung bezieht sich auf eine Vorrichtung zum Behandeln von Schüttgut, vorzugsweise von Saatgut, mit beschleunigten Elektronen. Das bevorzugte Anwendungsgebiet ist die phytosanitäre Behandlung von Saatgut gegen samenbürtige Schaderreger, die überwiegend in der Samenschale der Samenkörner angesiedelt sind. Weitere Anwendungsgebiete sind die Oberflächensterilisation von Granulaten und Pulvern, die chemische Oberflächenaktivierung sowie die Durchführung anderer strahlenchemischer Prozesse an Schüttgut.

### Stand der Technik

Es sind verschiedene Verfahren und die entsprechenden Vorrichtungen zum Beaufschlagen von Schüttgut mit beschleunigten Elektronen in verschiedenen Ausführungen - angepasst an das zu behandelnde Schüttgut - bekannt.

So wird in einer evakuierten Kammer durch gegenüberliegendes Anordnen zweier Elektronenbeschleuniger ein Elektronenfeld mit entgegengesetzten Geschwindigkeitskomponenten der Elektronen erzeugt, durch welches das Schüttgut im freien Fall in einem ausgedehnten transparenten Strom geführt wird (DD 291 702 A5). Zur Elektronenbehandlung wird das Schüttgut über Zellenradschleusen in die Kammer eingeschleust und nach dem Elektronenstrahlprozess wieder ausgeschleust. Der Nachteil solcher Vorrichtungen ist jedoch der hohe apparative Aufwand für das Erzeugen des Elektronenfeldes, da mindestens zwei Elektronenbeschleuniger erforderlich sind, und der hohe vakuumtechnische Aufwand.

Es ist außerdem bekannt, ein Elektronenfeld mit entgegengesetzten Geschwindigkeitskomponenten dadurch zu erzeugen, dass der Elektronenstrahl, nachdem er den Strom der Schüttgutteilchen passiert hat, durch eine magnetische Umlenkung auf den Teilchenstrom zurückgelenkt wird. Vorrichtungen dieser Art vermeiden den Aufwand für einen zweiten Elektronenbeschleuniger. Der Nachteil dieses Verfahrens besteht jedoch darin, dass durch den relativ langen Weg, den der Elektronenstrahl in der Prozesskammer durchläuft, ein wesentlich besseres Vakuum benötigt wird, was bezüglich der Vakuumerzeugung einen noch höheren apparativen Aufwand erfordert.

Es sind auch Verfahren und Vorrichtungen bekannt, die mit zwei einander gegenüberliegenden Elektronenbeschleunigern arbeiten, wobei die Elektronen über ein Strahlaustrittsfenster an Atmosphärendruck austreten (DE 44 34 767 C1). Das Schüttgut wird dabei ebenfalls im freien Fall durch das Elektronenfeld geführt. Bei dieser Lösung entfällt der Aufwand zum sonst erforderlichen Evakuieren der Prozesskammer. Dennoch verbleibt der Nachteil des hohen apparativen Aufwandes durch den notwendigen Einsatz von mindestens zwei Elektronenbeschleunigern.

Es ist weiterhin bekannt, pulverförmige und körnige Materialien an Atmosphärendruck mit Elektronen zu beaufschlagen, wobei nur ein Elektronenbeschleuniger zum Einsatz gelangt und die zu bestrahlenden Teilchen in einem Gasstrom durch das Elektronenfeld getragen werden (WO 98/43274 A1). Der Gasstrom mit den zu bestrahlenden Teilchen wird durch einen rechteckigen Kanal geführt, der an einer Seite mit einer 25 µm dicken Aluminiumfolie verschlossen ist, durch welche die Elektronen nach ihrer Ausschleusung über eine 13 µm dicke Titanfensterfolie und Durchlaufen der Distanz bis zum Bestrahlungskanal eindringen. Der Aluminiumfolie gegenüberliegend wird der rechteckige Kanal durch eine ebene Platte aus einem Werkstoff hoher Ordnungszahl gebildet. Nach Durchdringen des Kanalquerschnitts werden die Elektronen von dieser Platte zu einem gewissen Anteil rückgestreut. Die rückgestreuten Elektronen haben eine der ursprünglichen Einfallsrichtung der Elektronen entgegengerichtete Geschwindigkeitskomponente und ermöglichen, dass auch die bezüglich der ursprünglichen Einfallsrichtung der Elektronen abgewandte Seite der Teilchen einem Elektronenbeschuss ausgesetzt ist.

Von Nachteil ist, dass die Intensität der Bestrahlung durch die rückgestreuten Elektronen wesentlich niedriger ist als die Intensität der Bestrahlung durch die unmittelbar aus dem Strahlaustrittsfenster austretenden Elektronen, was zu einer ungleichmäßigen Bestrahlung der einzelnen Teilchen führt. Nachteilig ist auch, dass die zum Tragen der Teilchen erforderliche Gasgeschwindigkeit mit steigendem Verhältnis von Masse zur Oberfläche der transportierten Teilchen stark ansteigt. Somit würden für größerkörnige Schüttgüter - wie z. B. Weizen oder Mais - sehr hohe Gasströmungsgeschwindigkeiten erforderlich werden. Bei diesen hohen Geschwindigkeiten würden die im Elektronenfeld übertragbaren Energiedosen auf sehr kleine, für zahlreiche Anwendungen wesentlich zu geringe Werte begrenzt werden. Ein weiterer Nachteil dieser bekannten Lösung besteht darin, dass die Elektronen nach dem Austritt aus dem Elektronenbeschleuniger noch zusätzlich die den rechteckigen Kanal verschließende Aluminiumfolie durchdringen müssen, bevor sie auf die zu behandelnden Teilchen treffen. Dadurch erleiden die Elektronen einen zusätzlichen unerwünschten Energieverlust.

In DE 199 42 142 A1 ist schließlich eine Vorrichtung offenbart, bei der Schüttgut im mehrfachen freien Fall an einer Elektronenstrahleinrichtung vorbeigeführt und mit beschleunigten Elektronen beaufschlagt wird. Aufgrund des Mehrfachdurchlaufs, verbunden mit einer zwischenzeitlichen Durchmischung des Schüttguts, ist die Wahrscheinlichkeit bei dieser Ausführungsform sehr hoch, dass die Partikel des Schüttgutes allseitig mit beschleunigten Elektronen beaufschlagt werden. Der Mehrfachdurchlauf erfordert allerdings einen hohen Zeitaufwand bei der Durchführung des Behandlungsprozesses.

### Aufgabenstellung

Der Erfindung liegt daher das technische Problem zugrunde, eine Vorrichtung zum Beaufschlagen von Schüttgut mit beschleunigten Elektronen zu schaffen, mittels der die Nachteile des Standes der Technik überwunden werden können. Insbesondere soll die Vorrichtung gegenüber dem Stand der Technik kompaktere Abmaße ermöglichen und trotzdem einen hohen Durchsatz von zu behandelndem Schüttgut zulassen.

Die Lösung des technischen Problems ergibt sich durch Gegenstände mit den Merkmalen des Patentanspruchs 1. Weitere vorteilhafte Ausgestaltungen der Erfindung ergeben sich aus den abhängigen Patentansprüchen.

Ein wesentliches Merkmal einer erfindungsgemäßen Vorrichtung besteht darin, dass diese ringförmig ausgebildet ist, bei der die Elektronen in Richtung des Ringinneren beschleunigbar sind. Auf diese Weise kann ein Substrat, welches durch das Ringinnere der Vorrichtung geführt wird, in einem Bestrahlungsdurchgang vollumfänglich bezüglich eines Substratquerschnitts mit beschleunigten Elektronen beaufschlagt werden. An dieser Stelle sei ausdrücklich darauf verwiesen, dass der Begriff "ringförmig" im Erfindungssinn bei allen nachfolgend beschriebenen ringförmigen Vorrichtungen und Bauelementen nicht nur auf einen Ring in Kreisform begrenzt ist, sondern dass sich der Begriff "ringförmig" im Erfindungssinn lediglich auf einen schleifenförmig in sich geschlossenen Gegenstand bezieht, wobei der schleifenförmig in sich geschlossenen Gegenstand einen Freiraum in seinem Querschnitt vollständig umschließt und wobei Schüttgut durch diesen Freiraum im Inneren des Ringes hindurchgeführt werden kann. Dabei ist der von einem Ring vollständig umschlossene Querschnitt des Freiraumes zwar bei einer bevorzugten Ausführungsform der Erfindung kreisförmig ausgebildet, kann aber im weitesten Erfindungssinn auch jede andere geometrische Form aufweisen.

Der ringförmige Elektronenstrahlerzeuger einer erfindungsgemäßen Vorrichtung ist bei einer Ausführungsform derart angeordnet, dass die Ringachse des ringförmigen Elektronenstrahlerzeugers senkrecht ausgerichtet ist. Alternativ sind auch Ausführungsvarianten möglich, bei der die Ringachse bis zu 45° von der Senkrechten abweicht. Vorzugsweise weicht die Ringachse des ringförmigen Elektronenstrahlerzeugers nicht mehr als 10° von der Senkrechten ab. Bei einem derart angeordneten ringförmigen Elektronenstrahlerzeuger ist es möglich, Schüttgut im freien Fall durch das Ringinnere des ringförmigen Elektronenstrahlerzeugers hindurchfallen zu lassen und während des freien Falls durch das Ringinnere das Schüttgut mit beschleunigten Elektronen zu beaufschlagen.

Wenn sichergestellt werden soll, dass die Schüttgutpartikel möglichst vollumfänglich mit beschleunigten Elektronen beaufschlagt werden, wird der durch das Ringinnere des ringförmigen Elektronenstrahlerzeugers fallende Schüttgutstrom vorzugsweise derart ausgebildet, dass die Schüttgutpartikel vereinzelt durch das Ringinnere hindurchfallen. Eine erfindungsgemäße Vorrichtung umfasst daher neben einem ringförmigen Elektronenstrahlerzeuger auch noch eine oberhalb des ringförmigen Elektronenstrahlerzeugers angeordnete Einrichtung zum Vereinzeln von Schüttgutpartikeln, deren untere Begrenzung mindestens eine Öffnung aufweist, aus der Schüttgutpartikel heraus- und von dort durch den vom Elektronenstrahlerzeuger geformten Ring hindurchfallen. Die Einrichtung zum Vereinzeln von Schüttgutpartikeln kann beispielsweise ein Gefäß zur Aufnahme von Schüttgutpartikeln umfassen, wobei der Boden des Gefäßes mindestens eine Öffnung aufweist, aus der die Schüttgutpartikel herausfallen können. Vorzugsweise wird die Öffnung so groß dimensioniert, dass lediglich immer nur ein Schüttgutpartikel nach dem anderen durch die Öffnung im Boden des Gefäßes hindurchfallen kann. Wenn die Schüttgutpartikel eine annähernd gleiche Größe aufweisen, kann die Größe der Öffnung im Boden des Gefäßes derart ausgebildet sein, dass deren Durchmesser kleiner ist als der zweifache mittlere Durchmesser der Schüttgutpartikel. Bei Verwendung eines kreisrunden ringförmigen Elektronenstrahlerzeugers, dessen Ringachse senkrecht ausgerichtet ist, wird die Einrichtung zum Vereinzeln von Schüttgutpartikeln vorzugsweise derart oberhalb des Elektronenstrahlerzeugers angeordnet, dass sich die Öffnung im Boden des Gefäßes zur Aufnahme von Schüttgutpartikeln auf der verlängerten Ringachse des Elektronenstrahlerzeugers befindet. Auf diese Weise fallen die Schüttgutpartikel mittig durch den Elektronenstrahlerzeuger und werden vollumfänglich mit einer zumindest annähernd gleichen Energiedosis beaufschlagt.

Bei einer solchen Vorrichtung, bei der immer nur ein Schüttgutpartikel nach dem anderen durch das Ringinnere des ringförmigen Elektronenstrahlerzeugers hindurchfällt, kann sichergestellt werden, dass die Schüttgutpartikel vollumfänglich mit beschleunigten Elektronen beaufschlagt werden. Der Durchsatz der mit Elektronen beaufschlagten Schüttgutpartikel ist jedoch nur gering.

Bei einer alternativen Ausführungsform weist die Bodenwandung eines Gefäßes zur Aufnahme und Vereinzelung von Schüttgutpartikeln eine oder mehrere Öffnungen auf, die nicht an der Position der verlängerten Ringachse des ringförmigen Elektronenstrahlerzeugers angeordnet sind. Ein weiteres Merkmal der Bodenwandung besteht darin, dass diese rotierbar oder innerhalb der horizontalen Ebene bewegbar angeordnet ist.

Bei einer weiteren alternativen Ausführungsform umfasst eine erfindungsgemäße Vorrichtung einen kreisrunden, ringförmigen Elektronenstrahlerzeuger, dessen Ringachse senkrecht ausgerichtet ist, sowie eine oberhalb des Elektronenstrahlerzeugers angeordnete Einrichtung zum Vereinzeln von Schüttgutpartikeln, die mehrere Öffnungen in der Bodenwandung aufweist. Die Öffnungen in der Bodenwandung sind vorzugsweise radialsymmetrisch, mit jeweils gleichem Abstand auf einer kreisförmigen Bahn um die verlängerte Achse des Elektronenstrahlerzeugers herum angeordnet. Dabei ist der Radius der kreisförmigen Bahn, auf der die Öffnungen in der Bodenwandung eingebracht sind, kleiner als der Innenradius des ringförmigen Elektronenstrahlerzeugers, so dass auch alle aus den Öffnungen der Bodenwandung fallenden Schüttgutpartikel durch die Ringöffnung des ringförmigen Elektronenstrahlerzeugers hindurchfallen. Vorzugsweise sind die Öffnungen in der Bodenwandung der Einrichtung zum Vereinzeln der Schüttgutpartikel so groß ausgebildet, dass immer nur ein Schüttgutpartikel nach dem anderen durch eine Öffnung hindurchfallen kann.

Auf diese Weise wird ein transparenter, ringförmiger Schüttgutstrom - bestehend aus fallenden Schüttgutpartikeln - erzeugt, der durch die Ringöffnung des ringförmigen Elektronenstrahlerzeugers hindurchfällt und dabei mit beschleunigten Elektronen beaufschlagt wird. Transparent in Hinsicht auf den ringförmigen Strom fallender Schüttgutpartikel bedeutet, dass benachbarte fallende Schüttgutpartikel jeweils mit einem Maß voneinander beabstandet sind, so dass auch immer einige vom ringförmigen Elektronenstrahlerzeuger beschleunigte Elektronen zwischen den fallenden Schüttgutpartikeln hindurchgelangen können, welche dann auf der gegenüberliegenden Seite des ringförmigen, fallenden Schüttgutstroms auf die zur Ringachse ausgerichtete Rückseite von Schüttgutpartikeln auftreffen, wodurch auch bei einer solchen Vorrichtung die fallenden Schüttgutpartikel vollumfänglich mit beschleunigten Elektronen beaufschlagt werden.

Alternativ kann ein transparenter, ringförmiger Schüttgutstrom - bestehend aus fallenden, vereinzelten Schüttgutpartikeln - erzeugt werden, indem in die Bodenwandung einer oberhalb eines ringförmigen Elektronenstrahlerzeugers angeordneten Einrichtung zum Vereinzeln von Schüttgutpartikeln eine ringförmige Öffnung eingebracht wird. Dabei wird die Ringöffnung vorzugsweise radialsymmetrisch um die verlängerte Ringachse des ringförmigen Elektronenstrahlerzeugers herum ausgebildet. Die Spaltbreite der ringförmigen Öffnung wird dabei vorzugsweise so groß dimensioniert, dass bezüglich der Spaltbreite nur ein Schüttgutpartikel hindurchpasst. Auf diese Weise wird ebenfalls ein transparenter, ringförmiger Schüttgutstrom aus fallenden Schüttgutpartikeln mit einer Ringbreite von einem Schüttgutpartikel erzeugt.

Ebenfalls vorteilhaft ist es, wenn bei allen zuvor aufgeführten Ausführungsbeispielen mindestens ein Bauteil der Einrichtung zum Vereinzeln von Schüttgutpartikeln mit einer Vibration beaufschlagt wird, damit die mindestens eine Öffnung in der Bodenwandung der Einrichtung nicht von Schüttgutpartikeln verstopft wird. So kann zum Beispiel die Bodenwandung der Einrichtung zum Vereinzeln von Schüttgutpartikeln oder deren seitliche Wandungen mit einer Schwingung beaufschlagt und somit in Vibrationen versetzt werden. Hierfür einsetzbare Schwingungserzeuger, die mit einem Bauteil der Einrichtung zum Vereinzeln von Schüttgutpartikeln in mechanischen Kontakt gebracht werden, sind bekannt.

### Ausführungsbeispiel

Die Erfindung wird nachfolgend anhand von Ausführungsbeispielen näher erläutert. Die Fig. zeigen:
- Fig. 1: eine schematische und perspektivische Schnittdarstellung eines ringförmigen Elektronenstrahlerzeugers mit Poloidalspulen;
- Fig. 2: eine schematische und perspektivische Schnittdarstellung eines alternativen ringförmigen Elektronenstrahlerzeugers mit anodischen drahtförmigen Elektroden;
- Fig. 3: eine schematische Schnittdarstellung einer erfindungsgemäßen Vorrichtung im Querschnitt;
- Fig. 4a: eine schematische Schnittdarstellung einer alternativen erfindungsgemäßen Vorrichtung im Querschnitt;
- Fig. 4b: eine schematische Darstellung eines Gefäßes zur Aufnahme von Schüttgutpartikeln der Vorrichtung aus Fig. 4a als Draufsicht;
- Fig. 5a: eine schematische Schnittdarstellung einer weiteren alternativen erfindungsgemäßen Vorrichtung im Querschnitt;
- Fig. 5b: eine schematische Darstellung eines Gefäßes zur Aufnahme von Schüttgutpartikeln der Vorrichtung aus Fig. 5a als Draufsicht;
- Fig. 6: eine schematische Schnittdarstellung einer erfindungsgemäßen Vorrichtung mit Elektronenreflektor;
- Fig. 7: eine schematische Schnittdarstellung eines alternativen Gefäßes zur Aufnahme von Schüttgutpartikeln.

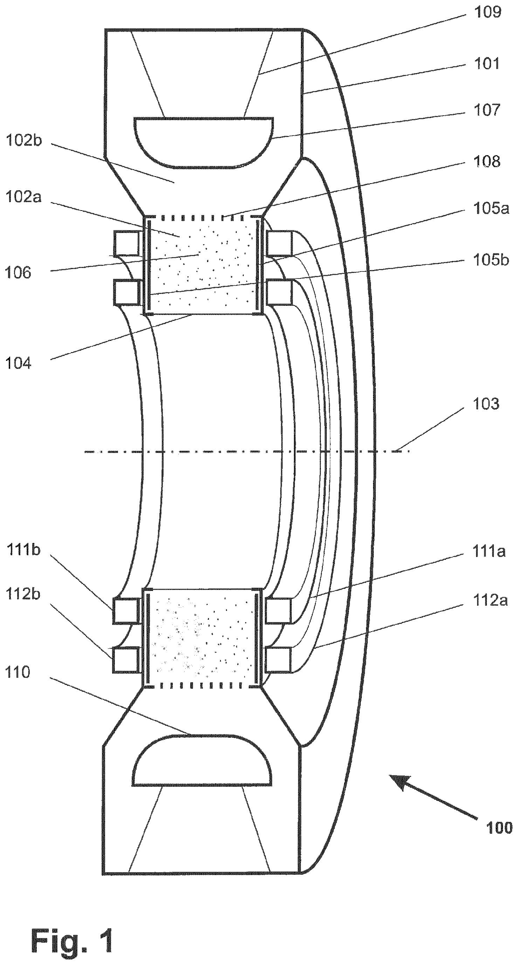

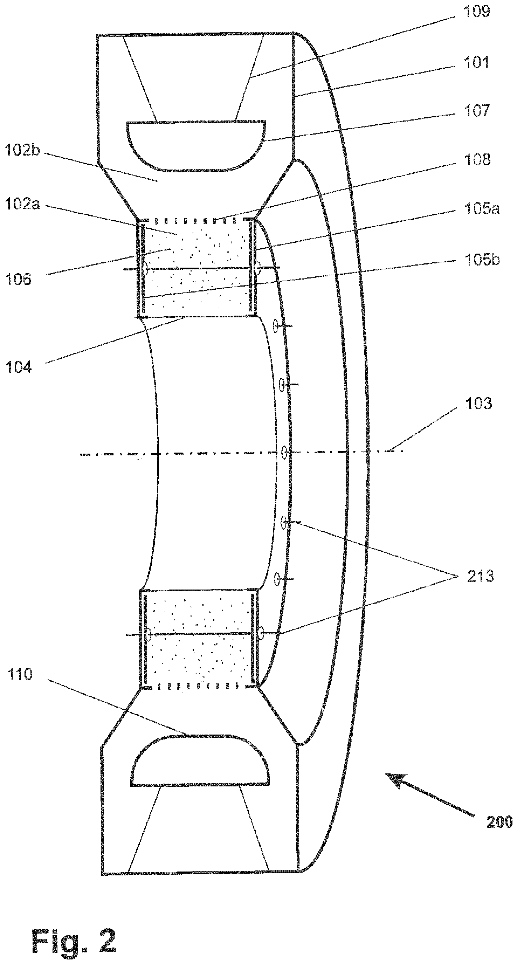

In den Fig. 1 und 2 sind zunächst einmal ringförmige Elektronenstrahlerzeuger schematisch dargestellt, die bei einer erfindungsgemäßen Vorrichtung verwendet werden können. Zum besseren Verständnis seien an dieser Stelle noch die Begriffe "Ringzylinder" und "Ringscheibe" in Bezug auf einen ringförmigen Gegenstand definiert. Wird der Innenradius eines kreisförmigen Ringes von seinem Außenradius subtrahiert, dann ergibt sich ein Maß. Ist dieses Maß kleiner als die Ausdehnung des Ringes in Richtung seiner Ringachse, so ist der Ring als Ringzylinder ausgebildet. Ist dieses Maß hingegen größer als die Ausdehnung des Ringes in Richtung seiner Ringachse, so ist der Ring als Ringscheibe ausgebildet.

Fig. 1 zeigt einen ringförmigen Elektronenstrahlerzeuger 100 als schematische perspektivische Schnittdarstellung. Ein solcher Elektronenstrahlerzeuger umfasst zunächst ein ringförmiges Gehäuse 101, welches zumindest in einem Bereich einen evakuierbaren Raum 102 begrenzt, der in die evakuierbaren Räume 102a und 102b unterteilt ist. Dieser evakuierbare Raum 102 ist aufgrund der Gehäuseform ebenfalls ringförmig. Im Ausführungsbeispiel von Fig. 1 ist das Gehäuse 101 radialsymmetrisch um eine Ringachse 103 ausgebildet. Alle nachfolgend beschriebenen, zum Elektronenstrahlerzeuger 100 zugehörigen und als ringförmig bezeichneten Bauelemente sind ebenfalls radialsymmetrisch und weisen ein und dieselbe Ringachse 103 auf. An der Ringinnenseite des Gehäuses 101 ist das Gehäuse 101 als Elektronenaustrittsfenster 104 in Form eines Ringzylinders ausgebildet, d. h. in Austrittsrichtung der Elektronen betrachtet weist das Elektronenaustrittsfenster 104 eine Oberflächensenkrechte auf, die zum Ringinneren und bei einem kreisförmigen Ringzylinder wie beim Elektronenaustrittsfenster 104 zur Ringachse 103 ausgerichtet ist. Durch mindestens einen in Fig. 1 nicht dargestellten Einlass im Gehäuse 101 wird ein Arbeitsgas in den evakuierbaren Raum 102 eingelassen und mittels mindestens einer in Fig. 1 ebenfalls nicht dargestellten Pumpeinrichtung ein Vakuum im evakuierbaren Raum 102 im Bereich von 0,5 Pa bis 1,5 Pa und bevorzugt im Bereich von 0,9 Pa bis 1,1 Pa aufrechterhalten.

Der ringförmige Elektronenstrahlerzeuger weist ferner mindestens eine erste Kathode und mindestens eine erste Anode auf, zwischen denen mittels einer ersten angelegten elektrischen Spannung, die von einer ersten Stromversorgungseinrichtung bereitgestellt wird, ein Glimmentladungsplasma im evakuierbaren Raum 102 erzeugbar ist. Im Ausführungsbeispiel wurden zwei als Ringscheibe ausgebildete erste Kathoden 105a und 105b verwendet, die im Raum 102a gegenüberliegend in der Nähe der seitlichen Innenwandungen des Gehäuses 101 angeordnet sind. Beim Elektronenstrahlerzeuger 100 wurde ferner das Gehäuse 101 als erste Anode geschaltet, wobei das Gehäuse 101 gleichzeitig das elektrische Massepotenzial des Elektronenstrahlerzeugers 100 aufweist. Alternativ kann aber auch mindestens eine separate Elektrode als erste Anode geschaltet werden, die vom Gehäuse 101 elektrisch isoliert ist. Die ringförmigen ersten Kathoden 105a und 105b sind mit einem derart geringen Maß von den jeweils angrenzenden Wandungen des Gehäuses 101 beabstandet, dass infolge einer Dunkelfeldabschirmung keine elektrische Entladung zwischen den Kathoden 105 und den unmittelbar angrenzenden Wandungen des als erste Anode geschalteten Gehäuses 101 ausgebildet wird. Eine zwischen dem als erste Anode geschalteten Gehäuse 101 und den ersten Kathoden 105 angelegte erste elektrische Spannung führt somit dazu, dass eine Glimmentladung zwischen einer ersten Kathode 105a, 105b und einer jeweils gegenüberliegenden Wandung des Gehäuses 101 ausgebildet wird. Ein auf diese Weise erzeugtes Plasma 106 füllt somit den Raum 102a zwischen den beiden ersten Kathoden 105a und 105b aus.

Ein ringförmiger Elektronenstrahlerzeuger umfasst des Weiteren mindestens eine zweite Kathode und mindestens eine zweite Anode, zwischen denen mittels einer zweiten Stromversorgungseinrichtung eine zweite elektrische Spannung geschaltet ist. Beim Elektronenstrahlerzeuger 100 ist eine ringförmige Kathode 107 als zweite Kathode und eine ringförmige und gleichzeitig gitterförmige Anode 108 als zweite Anode ausgebildet.

Die zweite Kathode stellt bei einem ringförmigen Elektronenstrahlerzeuger die Kathode zum Emittieren von Sekundärelektronen dar, welche anschließend beschleunigt werden, und weist hierfür ein elektrisches Hochspannungspotenzial von mindestens -50 kV, bevorzugt im Bereich von -100 kV bis -300 kV, auf. Mittels eines Isolators 109 ist die zweite Kathode 107 elektrisch gegenüber dem Gehäuse 101 isoliert.

Bei der in Fig. 1 beschriebenen Ausführungsform eines ringförmigen Elektronenstrahlerzeugers weisen die zweite Anode 108 und die ersten Kathoden 105a, 105b das gleiche elektrische Potenzial auf, das nur wenige Prozent des Spannungspotenzials der zweiten Kathode 107 beträgt und vorzugsweise aus dem Bereich von -0,5 kV bis -5 kV ausgewählt ist. Alternativ können die zweite Anode und die erste Kathode auch unterschiedliche elektrische Potenziale aufweisen, wobei jedoch die beiden Spannungspotenziale eine wesentlich geringere Spannungsdifferenz zur ersten Anode als zur zweiten Kathode aufweisen.

Aus dem Plasma 106 im Raum 102a driften Ionen durch die gitterförmige zweite Anode 108 in Richtung der zweiten Kathode 107. Dort treffen die Ionen auf einen Oberflächenbereich 110 der zweiten Kathode 107, dessen Oberflächensenkrechte zum Ringinneren des Gehäuses und bei einem radialsymmetrischen Gehäuse, wie Gehäuse 101, zur Ringachse 103 ausgerichtet ist. Beim Auftreffen der Ionen auf den Oberflächenbereich 110 haben die Ionen somit eine Potenzialdifferenz durchfallen, die weitgehend der Beschleunigungsspannung des Elektronenstrahlerzeugers 100 entspricht. Bei ihrem Auftreffen wird die Energie der Ionen in einer sehr dünnen Randschicht der Kathode 107 im Oberflächenbereich 110 frei, was zur Auslösung von Sekundärelektronen führt. Bei den zuvor genannten elektrischen Spannungen an der zweiten Kathode 107 ist das Verhältnis zwischen ausgelösten Elektronen und auftreffenden Ionen in der Größenordnung 10 angesiedelt, was diese Art des Erzeugens beschleunigter Elektronen sehr effizient macht. Die entstandenen Sekundärelektronen werden vom anliegenden elektrischen Feld stark beschleunigt und durchfliegen die in Form eines Ringzylinders ausgebildete gitterförmige Anode 108 und das Plasma 106 im Raum 102a. Nach dem Durchqueren des Elektronenaustrittsfensters 104, das beispielsweise als dünne Metallfolie ausgeführt sein kann, dringen die Elektronen in den vom ringförmigen Gehäuse 101 umschlossenen Freiraum vor, in dem ein höherer Druck als im Raum 102 herrschen kann und durch den ein mit Elektronen zu beaufschlagendes Schüttgut hindurchfallen kann. Als Material für das Elektronenaustrittsfenster 104 können alle aus dem Stand der Technik für ein Elektronenaustrittsfenster bekannten Materialien, wie beispielsweise Titan, verwendet werden. Außerdem ist es zum Zwecke einer höheren mechanischen Stabilität des Elektronenaustrittsfensters 104 vorteilhaft, dieses mit einem Stützgitter zu versehen, wie es ebenfalls aus dem Stand der Technik bekannt ist. Aufgrund der ringförmigen Gestalt aller zuvor genannten Bauteile eines ringförmigen Elektronenstrahlerzeugers wird mit diesem ein in sich geschlossenes, ringförmiges Band beschleunigter Elektronen erzeugt, wobei die Bewegungsrichtung der beschleunigten Elektronen auf den vom Gehäusering eingeschlossenen Freiraum ausgerichtet ist. Der Freiraum, der vom Gehäusering umschlossen wird und durch den ein zu behandelndes Schüttgut hindurchfällt, wird nachfolgend auch als Behandlungsraum bezeichnet. Bei einem radialsymmetrischen ringförmigen Elektronenstrahlerzeuger, wie Elektronenstrahlerzeuger 100, ist die Bewegungsrichtung der beschleunigten Elektronen vorzugsweise auf die Ringachse 103 ausgerichtet. Vereinzeltes Schüttgut, welches durch das Ringinnere des Gehäuses eines solchen ringförmigen Elektronenstrahlerzeugers fällt, kann somit vollumfänglich in einem Durchlauf mit beschleunigten Elektronen beaufschlagt werden.

Der Vollständigkeit halber sei an dieser Stelle erwähnt, dass ein ringförmiger Elektronenstrahlerzeuger auch eine Einrichtung zum Kühlen aufweist, wie es auch bei anderen Einrichtungen zum Erzeugen beschleunigter Elektronen aus dem Stand der Technik bekannt ist. So kann diese Einrichtung zum Kühlen eines ringförmigen Elektronenstrahlerzeugers beispielsweise Kühlkanäle umfassen, die sich innerhalb des Isolators 109 erstrecken und durch die ein Kühlmedium strömt.

Die zweite Anode 108, welche bei einem ringförmigen Elektronenstrahlerzeuger bevorzugt als gitterförmiger Ringzylinder ausgebildet ist und welche die räumliche Grenze zwischen den evakuierbaren Räumen 102a und 102b darstellt, erfüllt zwei wesentliche Aufgaben. Zum einen bewirkt sie aufgrund ihrer Spannungsdifferenz gegenüber der zweiten Kathode 107 eine Beschleunigung der emittierten Sekundärelektronen. Aufgrund des Sachverhaltes, dass die ringförmige Gitterstruktur der zweiten Anode 108 parallel zur Sekundärelektronen emittierenden Oberfläche 110 der zweiten Kathode 107 ausgebildet ist, wird ein elektrisches Feld derart ausgebildet, dass auch die Bahnen der beschleunigten Sekundärelektronen weitgehend parallel verlaufen. Zum anderen schirmt die zweite Anode 108 das Plasma vom Spannungspotenzial der zweiten Kathode 107 ab; verhindert dadurch das Abdriften zu vieler Ionen in Richtung zweiter Kathode 107 und trägt somit zum Aufrechterhalten des Plasmas 106 im Raum 102a bei.

Für das Aufrechterhalten des Plasmas 106 sind jedoch noch weitere Maßnahmen erforderlich. Der relativ niedrige Druck von etwa 1 Pa im Raum 102a ermöglicht nur das Generieren einer relativ geringen Anzahl von niederenergetischen Elektronen infolge der Glimmentladung zwischen den ersten Kathoden 105 und des als erste Anode geschalteten Gehäuses 101. Das Driften dieser niederenergetischen Elektronen auf direktem Wege zwischen einer ersten Kathode 105 und der ersten Anode würde nur zu einer ungenügenden Anzahl von Stoßreaktionen mit Inertgaspartikeln und somit zum Erzeugen von Ionen führen, die zum Aufrechterhalten des Plasmas 106 nicht hinreichend wären. Es sind somit Maßnahmen notwendig, die den Weg der niederenergetischen Elektronen im Raum 102a verlängern und somit deren Stoßwahrscheinlichkeit und -häufigkeit erhöhen.

Eine solche Maßnahme ist in Fig. 1 in Form von ringförmigen Magnetspulen 111a, 111b, 112a, 112b dargestellt, die jeweils ein Magnetfeld mit poloidal verlaufenden Magnetfeldlinien erzeugen. Derartige Magnetspulen werden daher auch als Poloidalspulen bezeichnet. Die Magnetspulen 111a, 111b, 112a, 112b sind in zwei Paaren gegenüberliegend und außerhalb des Gehäuses 101 auf Höhe der ersten Kathoden 105 derart angeordnet, dass ihr Magnetfeld den Raum 102a durchdringt. Dabei weisen die Magnetspulen 111a und 111b identische Ringradien auf, die größer sind als die identischen Ringradien der Magnetspulen 112a und 112b. Die poloidal verlaufenden Magnetfeldlinien im Raum 102a zwingen die niederenergetischen Elektronen auf gekrümmte Bahnen mit energieabhängigem Gyrationsradius, was die Driftstrecke und dadurch auch die Verweildauer der niederenergetischen Elektronen im Raum 102 und somit die Anzahl Ionen erzeugender Stoßereignisse erhöht.

Die Magnetfelder der Magnetspulen 111a, 111b, 112a, 112b beeinflussen jedoch nicht nur die Bahnen der niederenergetischen Elektronen, sondern auch die Bahnen der hochenergetischen, aus der zweiten Kathode 107 emittierten und anschließend beschleunigten, sowie den Raum 102a durchfliegenden Elektronen. Ohne die Magnetfelder der Magnetspulen 111a, 111b, 112a, 112b würden die meisten beschleunigten, hochenergetischen Elektronen in einem senkrechten Winkel bzw. in einem Winkel nahe dem senkrechten Winkel durch das Elektronenaustrittsfenster 104 hindurchtreten. Die Magnetfelder der Magnetspulen 111a, 111b, 112a, 112b verändern jedoch diesen Durchtrittswinkel, was je nach Anwendungsfall von Vorteil oder Nachteil sein kann. Vorteilhaft ist dies beispielsweise beim Homogenisieren des Energieeintrags in Substrate mit gekrümmten Oberflächenbereichen oder zum Beaufschlagen von Oberflächenbereichen eines Substrates, die sich bei rein radialer Ausbreitungsrichtung der beschleunigten Elektronen in abgeschattet liegenden Oberflächenbereichen befinden würden.

Bei vielen Anwendungsfällen ist es jedoch vorteilhaft, wenn der Durchtritt von beschleunigten Elektronen durch das Elektronenaustrittsfenster 104 mit einem rechten Winkel zur Oberfläche des Elektronenaustrittsfensters 104 bzw. mit einem Winkel nahe dem rechten Winkel erfolgt. Außerdem führt jede Abweichung vom Normaleinfall zu einer höheren Absorption von Elektronen in der Folie des Elektronenaustrittsfensters 104 und insbesondere auch in der die Folie üblicherweise stützenden und kühlenden Gitterstruktur des Elektronenaustrittsfensters. Bei den Anwendungsfällen, bei denen die beschleunigten Elektronen möglichst senkrecht durch das Elektronenaustrittsfenster 104 hindurchtreten sollen, ist daher bei Benutzung von Magnetspulen 111a, 111b, 112a, 112b eine Korrektur der Flugbahn der beschleunigten Elektronen erforderlich.

Das Ablenken der Flugbahnen beschleunigter Elektronen infolge der Magnetfelder der Magnetspulen 111a, 111b, 112a, 112b kann beispielsweise mittels mindestens eines zusätzlichen Paares von gegenüberliegend angeordneten Poloidalspulen ausgeglichen werden, wobei das zusätzliche Paar von Poloidalspulen einen äußeren Radius aufweist, der noch kleiner ist als der innere Radius der Magnetspulen 111a, 111b. Dabei sind jedoch vorzugsweise die Innenradien aller drei ringförmigen Magnetspulenpaare größer als der Außenradius des zylinderförmigen Elektronenaustrittsfensters 104 und die Außenradien aller der ringförmigen Magnetspulenpaare kleiner als der Innenradius der gitterförmigen zweiten Anode 108. Auf diese Weise wird sichergestellt, dass sich die Magnetfelder der ringförmigen Magnetspulen innerhalb einer erfindungsgemäßen Vorrichtung hauptsächlich im Raum 102a erstrecken und dort zum Aufrechterhalten des Plasmas bzw. zur Korrektur der Flugbahn beschleunigter Elektronen beitragen.

Alternativ ist aber auch je nach Anwendungsfall eine andere Anordnung der Magnetspulen möglich. Förderliche Werte für Anzahl, Position und Erregung der Magnetspulen für einen jeweiligen Anwendungsfall lassen sich beispielsweise mittels Simulationsrechnungen finden. Eine weitere Lösung zum Verlängern der Driftbahnen von niederenergetischen Elektronen zwischen erster Kathode und erster Anode ist in Fig. 2 angegeben. In Fig. 2 ist ein alternativer ringförmiger Elektronenstrahlerzeuger 200 als schematische perspektivische Schnittdarstellung aufgezeigt, der zunächst identische Bauteile und Merkmale des Elektronenstrahlerzeugers 100 aus Fig. 1 umfasst, außer den Magnetspulen 111a, 111b, 112a, 112b. Statt der Magnetspulen weist Elektronenstrahlerzeuger 200 eine Anzahl von drahtförmigen Elektroden 213 auf, die sich durch den Raum 102a hindurch erstrecken und bei einem Gehäuse in Form eines kreisförmigen Ringes, wie Gehäuse 101, auf einem identischen Radius und mit gleichem Abstand zueinander um die Achse 103 herum angeordnet sind. Dabei werden die drahtförmigen Elektroden 213, die ein leicht positives Spannungspotenzial in einem Bereich von +0,5 kV bis +5,0 kV gegenüber dem Gehäuse 101 aufweisen können, elektrisch isoliert durch das Gehäuse 101 und die ersten Kathoden 105a, 105b hindurchgeführt. Aufgrund der drahtförmigen Elektroden 213 werden die niederenergetischen Elektronen im Raum 102a ebenfalls auf spiralförmige und somit verlängerte Bahnen gelenkt und damit das Plasma 106 aufrechterhalten.

Um die Entstehung von Ozon und Stickoxyden zu minimieren, ist die Spülung des Behandlungsraumes mit einem Edelgas empfehlenswert, wodurch gleichzeitig auch ein Kühleffekt am zu behandelnden Schüttgut und am Elektronenaustrittsfenster entsteht. In diesem Zusammenhang ist es weiterhin denkbar, den Energieeintrag in ein mit beschleunigten Elektronen zu beaufschlagendes Schüttgut, welches durch den Behandlungsraum fällt, durch geeignete Wahl oder Mischung unterschiedlicher Gase im Behandlungsraum zusätzlich zu beeinflussen, da die Energieabsorption und Elektronenstreuung auf der Gasstrecke zwischen Elektronenaustrittsfenster und der zu beaufschlagenden Oberfläche des Schüttgutes von der Massendichte der lokalen Atmosphäre abhängen.

Die Regelung der Hilfsentladung zwischen erster Kathode und erster Anode, über deren Intensität die Elektronenstrahlleistung eines ringförmigen Elektronenstrahlerzeugers geregelt werden kann, ist auf verschiedenen Wegen zu bewerkstelligen. Neben dem Gasfluss in den Elektronenstrahlerzeuger kommen noch die Hilfsentladungsspannung zwischen erster Kathode und erster Anode sowie der Strom durch die ringförmigen Magnetspulen als Stellgröße hierfür in Frage. Bei einer Ausführungsform mit drahtförmigen Elektroden 213 stehen der Druck im Raum 102 und die elektrische Spannung der drahtförmigen Elektroden 213 als komplementäre Stellgrößen zur Auswahl. Dabei ist anzumerken, dass die elektrischen Stellgrößen generell kürzere Zeitkonstanten aufweisen und somit einerseits einer schnellen Regelung dienen können sowie andererseits einen gepulsten Betriebsmodus ermöglichen.

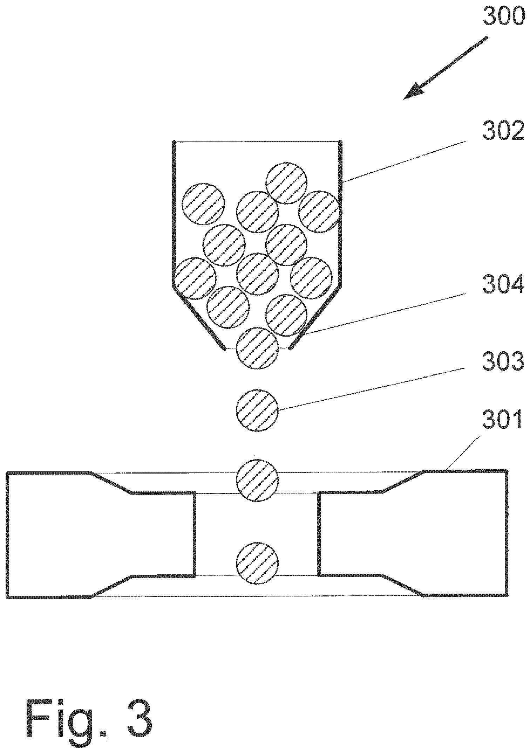

Fig. 3 zeigt eine schematische Schnittdarstellung einer erfindungsgemäßen Vorrichtung 300. Vorrichtung 300 umfasst einen ringförmigen Elektronenstrahlerzeuger 301, dessen Ringachse senkrecht ausgerichtet ist. Die Ringachse liegt auf der in Fig. 3 dargestellten Schnittebene. Oberhalb des Elektronenstrahlerzeugers 301 ist eine Vorrichtung zum Vereinzeln von Schüttgutpartikeln angeordnet. Die Vorrichtung zum Vereinzeln von Schüttgutpartikeln umfasst ein zylinderförmiges Gefäß 302, in welchem sich Schüttgutpartikel 303 befinden. Die Bodenwandung 304 des Gefäßes 302 ist kegelförmig ausgebildet, mit nach unten gerichteter Kegelspitze. Die Kegelspitze ist genau auf der verlängerten Ringachse des ringförmigen Elektronenstrahlerzeugers 301 positioniert. An der Kegelspitze weist die Bodenwandung 304 des Gefäßes 302 eine Öffnung der Größe auf, dass immer nur ein Schüttgutpartikel 303 nach dem anderen aus dem Gefäß 302 herausfallen und von dort mittig durch die Ringöffnung des ringförmigen Elektronenstrahlerzeugers 301 hindurchfallen kann. Während des Hindurchfallens durch die Ringöffnung des ringförmigen Elektronenstrahlerzeugers 301 werden die Schüttgutpartikel 303 vollumfänglich mit beschleunigten Elektronen aus dem Elektronenstrahlerzeuger 301 beaufschlagt. Die Einrichtung zum Vereinzeln der Schüttgutpartikel 303 umfasst ferner in Fig. 3 nicht dargestellte Mittel, mit denen die Schüttgutpartikel 303 im Gefäß 302 nachgefüllt werden. Des Weiteren kann die Einrichtung zum Vereinzeln von Schüttgutpartikeln mindestens einen in Fig. 3 ebenfalls nicht dargestellten Schwingungserzeuger umfassen, der einen mechanischen Kontakt mit dem Gefäß 302 aufweist, wodurch das Gefäß 302 vibriert.

Zuvor wurde dargelegt, dass die Größe der Öffnung in der Bodenwandung des Gefäßes 302 bevorzugt so dimensioniert ist, dass möglichst nur ein Schüttgutpartikel nach dem anderen durch die Öffnung hindurchfallen kann. Eine solche Ausführungsform ist insbesondere dann geeignet, wenn nur die Oberfläche von Schüttgutpartikeln mit beschleunigten Elektronen beaufschlagt werden, nicht aber ein gesamter Schüttgutpartikel vollständig von Elektronen durchdrungen werden soll, wie es beispielsweise bei der Behandlung von Saatgut der Fall ist.

Besteht eine auszuführende Aufgabe jedoch darin, Schüttgutpartikel vollständig mit beschleunigten Elektronen zu durchdringen, wie beispielsweise beim Modifizieren von Kunststoffpartikeln, kann die Öffnung im Gefäß 302 auch größer dimensioniert werden, so dass mehrere Schüttgutpartikel 303 gleichzeitig durch die Öffnung fallen können. Diese anwendungsbezogene Aussage hinsichtlich der Größe einer Öffnung in der Bodenwandung eines Gefäßes zur Aufnahme von Schüttgutpartikeln ist auch auf alle nachfolgend beschriebenen Ausführungsformen anwendbar.

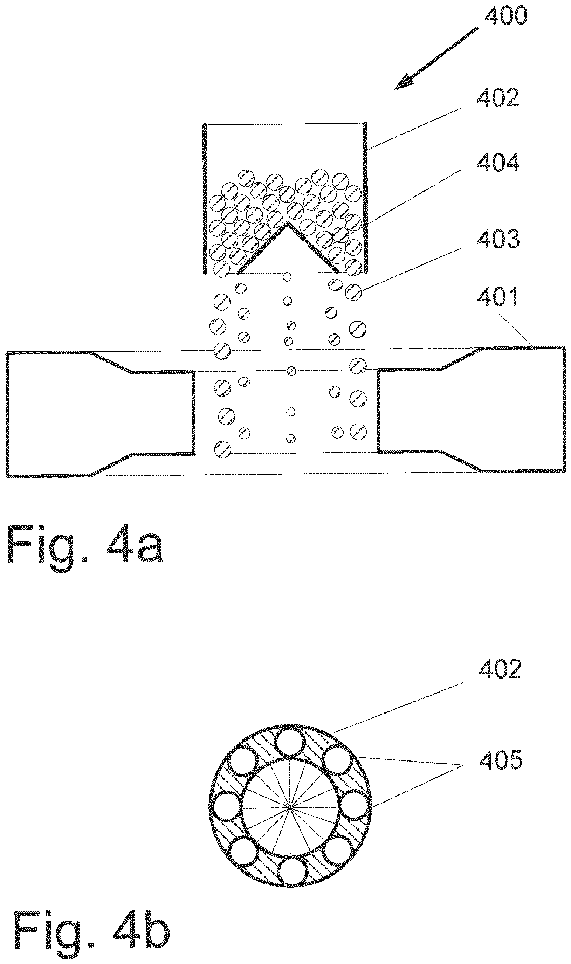

In Fig. 4a ist eine alternative erfindungsgemäße Vorrichtung 400 im Schnitt schematisch dargestellt. Vorrichtung 400 umfasst einen ringförmigen Elektronenstrahlerzeuger 401, dessen Ringachse senkrecht ausgerichtet ist. Die Ringachse liegt auf der in Fig. 4a dargestellten Schnittebene. Oberhalb des Elektronenstrahlerzeugers 401 ist eine Vorrichtung zum Vereinzeln von Schüttgutpartikeln angeordnet. Die Vorrichtung zum Vereinzeln von Schüttgutpartikeln umfasst ein zylinderförmiges Gefäß 402, in welchem sich Schüttgutpartikel 403 befinden. Das Gefäß 402 ist in Fig. 4b schematisch als Draufsicht dargestellt. Die Bodenwandung 404 des Gefäßes 402 ist in einem zentralen Bereich kegelförmig ausgebildet, mit nach oben gerichteter Kegelspitze. Die Kegelspitze ist genau auf der verlängerten Ringachse des ringförmigen Elektronenstrahlerzeugers 401 positioniert. Durch die Kegelform des zentralen Bereichs der Bodenwandung 404 werden die Schüttgutpartikel 403 in den unteren Bereich des Gefäßes 402 auf einen ringförmigen äußeren Bereich der Bodenwandung 404 gedrängt, der eine Vielzahl von Öffnungen 405 aufweist. Die Öffnungen 405 sind rotationssymmetrisch um die verlängerte Ringachse des ringförmigen Elektronenstrahlerzeugers 401 herum angeordnet und mit jeweils gleichem Maß von einer benachbarten Öffnung 405 beabstandet. Jede der Öffnungen 405 ist so groß, dass lediglich ein Schüttgutpartikel 403 nach dem anderen durch eine Öffnung 405 hindurchfallen kann.

Mittels dieser Einrichtung zum Vereinzeln von Schüttgutpartikeln wird ein transparenter, ringförmiger und rotationssymmetrisch um die Ringachse des Elektronenstrahlerzeugers 401 angeordneter Schüttgutstrom, bestehend aus fallenden Schüttgutpartikeln 403 erzeugt, der durch die Ringöffnung des ringförmigen Elektronenstrahlerzeugers 401 fällt und während des freien Falls mit beschleunigten Elektronen beaufschlagt wird.

Mittels solch einer Vorrichtung, bei der ein ringförmiger, transparenter Schüttgutstrom erzeugt wird, der mit beschleunigten Elektronen eines ringförmigen Elektronenstrahlerzeugers beaufschlagt wird, lassen sich kompaktere Anlagenabmaße realisieren als mit einer Vorrichtung aus dem Stand der Technik, bei der ein gleichlanger, transparenter, linearer Schüttgutstrom mit beschleunigten Elektronen aus Linearstrahlern heraus beaufschlagt wird. Ein weiterer Vorteil erfindungsgemäßer Vorrichtungen gegenüber dem Stand der Technik, bei dem zwei gegenüberliegend angeordnete Linearstrahler verwendet werden, besteht darin, dass nur ein Elektronenstrahlerzeuger verwendet wird. Somit werden bei einer erfindungsgemäßen Vorrichtung auch nur ein Satz von Peripheriegeräten (wie beispielsweise Vakuumpumpen und Stromversorgungseinrichtungen) benötigt, wohingegen beim Stand der Technik jeweils zwei Sätze von Peripheriegeräten benötigt werden.

Die Einrichtung zum Vereinzeln der Schüttgutpartikel 403 umfasst ferner in Fig. 4 nicht dargestellte Mittel, mit denen die Schüttgutpartikel 403 im Gefäß 402 nachgefüllt werden. Des Weiteren kann die Einrichtung zum Vereinzeln von Schüttgutpartikeln mindestens einen in Fig. 4 ebenfalls nicht dargestellten Schwingungserzeuger umfassen, der einen mechanischen Kontakt mit dem Gefäß 402 aufweist, wodurch das Gefäß 402 vibriert.

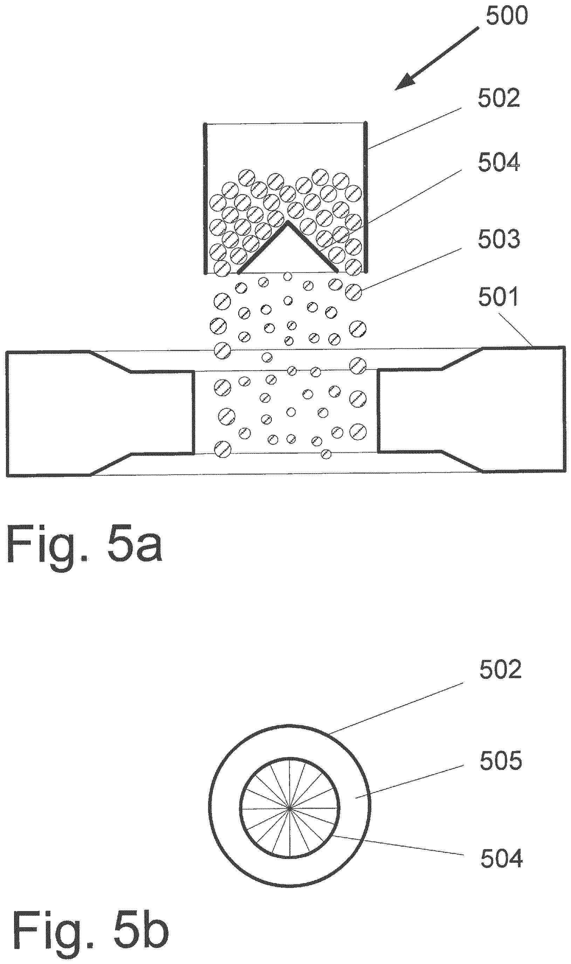

In Fig. 5a ist eine weitere alternative erfindungsgemäße Vorrichtung 500 im Schnitt schematisch dargestellt. Vorrichtung 500 umfasst einen ringförmigen Elektronenstrahlerzeuger 501, dessen Ringachse senkrecht ausgerichtet ist. Die Ringachse liegt auf der in Fig. 5a dargestellten Schnittebene. Oberhalb des Elektronenstrahlerzeugers 501 ist eine Vorrichtung zum Vereinzeln von Schüttgutpartikeln angeordnet. Die Vorrichtung zum Vereinzeln von Schüttgutpartikeln umfasst ein Gefäß zur Aufnahme von Schüttgutpartikeln 503. Das Gefäß besteht aus einer zylinderförmigen Seitenwandung 502 und einer kegelförmigen Bodenwandung 504, wobei zwischen der Seitenwandung 502 und der Bodenwandung 504 ein ringförmiger Spalt 505 ausgebildet ist. Das Gefäß ist in Fig. 5b schematisch als Draufsicht dargestellt. Die Kegelspitze der Bodenwandung ist nach oben gerichtet und genau auf der verlängerten Ringachse des ringförmigen Elektronenstrahlerzeugers 501 positioniert. Durch die Kegelform der Bodenwandung 504 werden die Schüttgutpartikel 503 im unteren Bereich des Zylinders 502 auf einen ringförmigen äußeren Bereich gedrängt, in dem die Schüttgutpartikel durch die Öffnung in Form des Ringspaltes 505 herausfallen. Der Ringspalt 405 ist rotationssymmetrisch um die verlängerte Ringachse des ringförmigen Elektronenstrahlerzeugers 401 herum angeordnet und weist eine Spaltbreite derart auf, dass bezüglich der Spaltbreite lediglich ein Schüttgutpartikel 503 nach dem anderen durch den Ringspalt 505 hindurchfallen kann. Auf die Länge des Ringspaltes 505 betrachtet können selbstverständlich mehrere Schüttgutpartikel 503 gleichzeitig durch den Ringspalt 505 hindurchfallen.

Mittels dieser Einrichtung zum Vereinzeln von Schüttgutpartikeln wird ein transparenter, ringförmiger und rotationssymmetrisch um die Ringachse des Elektronenstrahlerzeugers 501 angeordneter Schüttgutstrom, bestehend aus fallenden Schüttgutpartikeln 503, erzeugt, der durch die Ringöffnung des ringförmigen Elektronenstrahlerzeugers 501 fällt und während des freien Falls mit beschleunigten Elektronen beaufschlagt wird. Gegenüber einer Vorrichtung 400 aus Fig. 4a kann der mit einer Vorrichtung 500 erzeugte ringförmige Schüttgutstrom bezüglich seiner Ringwinkelausdehnung mit einer höheren Partikeldichte ausgebildet werden und ermöglicht somit einen höheren Schüttgutdurchsatz.

Auch die hierbei verwendete Einrichtung zum Vereinzeln der Schüttgutpartikel 503 umfasst in Fig. 5a nicht dargestellte Mittel, mit denen die Schüttgutpartikel 503 in den Zylinder 502 nachgefüllt werden. Des Weiteren kann diese Einrichtung zum Vereinzeln von Schüttgutpartikeln mindestens einen in Fig. 5a ebenfalls nicht dargestellten Schwingungserzeuger umfassen, der einen mechanischen Kontakt mit dem Zylinder 502 und/oder der Bodenwandung 504 aufweist, wodurch diese Bauteile vibrieren und dadurch einen kontinuierlichen, fallenden Schüttgutstrom gewährleisten.

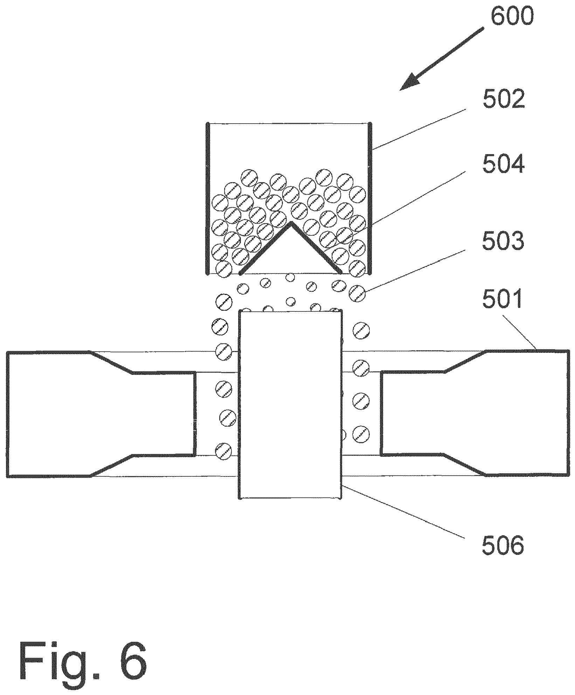

In Fig. 6 ist eine weitere erfindungsgemäße Vorrichtung 600 schematisch im Schnitt dargestellt, die zunächst einmal alle Merkmale der Vorrichtung 500 aus Fig. 5a aufweist. Zusätzlich weist die Vorrichtung 600 einen zylinderförmigen Elektronenreflektor 506 auf, der sich durch die Ringöffnung des ringförmigen Elektronenstrahlerzeugers 501 hindurch erstreckt. Dabei ist der zylinderförmige Elektronenreflektor 506 rotationssymmetrisch um die Ringachse des ringförmigen Elektronenstrahlerzeugers herum ausgebildet und weist einen Radius auf, der kleiner ist als der Radius des transparenten, ringförmigen Schüttgutstromes bestehend aus fallenden Schüttgutpartikeln 503. Mittels des zylinderförmigen Elektronenreflektors 506 können die beschleunigten Elektronen, die den transparenten ringförmigen Schüttgutstrom durchdringen, reflektiert und dadurch die fallenden Schüttgutpartikel 503 auf deren zur Ringachse ausgerichteten Rückseite mit beschleunigten Elektronen beaufschlagt werden.

Bei einer weiteren Ausführungsform ist der zylinderförmige Elektronenreflektor 506 bezüglich seiner Ringwinkelausdehnung in Segmente unterteilt, wobei jedes Segment elektrisch kontaktiert und mittels elektrischer Verbindungselemente mit einer Auswerteeinrichtung verbunden ist. Auf diese Weise wird für jedes Segment ein Wert erfasst, der der Anzahl der auf das Segment auftreffenden Elektronen entspricht. So kann beispielsweise für jedes Segment ein Wert für den elektrischen Strom erfasst werden. Eine solche Ausführungsform kann beispielsweise zur Qualitätssicherung verwendet werden, weil sich damit eine qualitative Aussage treffen lässt, ob verschiedene Ringwinkelsegmente des transparenten ringförmigen Schüttgutstromes mit einer zumindest annähernd gleichen Energiedosis beaufschlagt wurden. Wird bei einer solchen Vorrichtungskonfiguration auch noch ein ringförmiger Elektronenstrahlerzeuger verwendet, dessen zweite Kathode segmentiert ausgebildet ist oder ein Elektronenstrahlerzeuger, der drahtförmige Elektroden 213 aufweist, dann kann der ringförmige Elektronenstrahl des ringförmigen Elektronenstrahlerzeugers segmentweise derart geregelt werden, dass mögliche Unterschiede beim Elektronenstrom, der auf verschiedenen Segmenten des zylinderförmigen Elektronenreflektors erfasst wird, ausgeglichen werden. Damit kann eine gleichmäßige Beaufschlagung des ringförmigen Schüttgutstromes auf dem gesamten Ringumfang gewährleistet werden.

Solch ein zylinderförmiger Elektronenreflektor 506, ob segmentiert oder nicht segmentiert, kann bei allen Ausführungsformen der Erfindung, bei der ein ringförmiger, fallender Schüttgutstrom erzeugt wird, wie beispielsweise auch bei einer Ausführungsform gemäß Fig. 4a, verwendet werden.

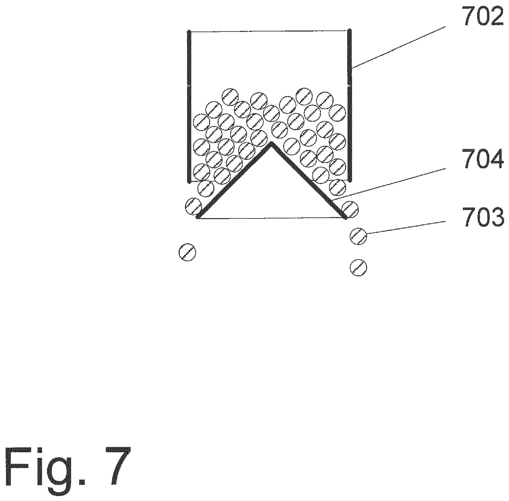

In Fig. 7 ist eine alternative Ausführungsform eines Gefäßes zur Aufnahme von Schüttgutpartikeln 703 schematisch im Schnitt dargestellt. Das Gefäß besteht aus einer zylinderförmigen Seitenwandung 702 und einer kegelförmigen Bodenwandung 704, wobei zwischen der Seitenwandung 702 und der Bodenwandung 704 ein ringförmiger Spalt ausgebildet ist. Die Kegelspitze der Bodenwandung 704 ist nach oben gerichtet und genau auf der verlängerten Ringachse eines zugehörigen ringförmigen Elektronenstrahlerzeugers positioniert. Durch die Kegelform der Bodenwandung 704 werden die Schüttgutpartikel 703 im unteren Bereich des Zylinders 702 auf einen ringförmigen äußeren Bereich gedrängt, in dem die Schüttgutpartikel durch die Öffnung in Form des Ringspaltes herausfallen. Die Bodenwandung 704 und/oder die zylinderförmige Seitenwandung 702 sind höhenverstellbar ausgebildet. Dadurch kann die Breite des Ringspaltes zwischen Seitenwandung 702 und Bodenwandung 702 eingestellt und an eine jeweilige Größe der Schüttgutpartikel 703 angepasst werden.

## Patentansprüche

1. Vorrichtung, umfassend mindestens einen Elektronenstrahlerzeuger (301) zum Generieren von beschleunigten Elektronen, mit denen Schüttgutpartikel (303) während des freien Falls entlang einer Senkrechten beaufschlagbar sind, **dadurch gekennzeichnet, dass**
a) der Elektronenstrahlerzeuger (301) ringförmig ausgebildet ist, bei dem die von einer ringförmigen Kathode emittierten und beschleunigten Elektronen aus einem Elektronenaustrittsfenster in Richtung Ringachse austreten;
b) der ringförmige Elektronenstrahlerzeuger (301) derart angeordnet ist, dass dessen Ringachse senkrecht oder mit einem Winkel von bis zu 45° abweichend von der Senkrechten ausgerichtet ist;
c) oberhalb des ringförmigen Elektronenstrahlerzeugers eine dazu gehörende Einrichtung zum Vereinzeln von Schüttgutpartikeln angeordnet ist, deren Bodenwandung (304) mindestens eine Öffnung aufweist, aus der Schüttgutpartikel (303) heraus- und von dort durch den vom Elektronenstrahlerzeuger (301) geformten Ring hindurchfallen können.

2. Vorrichtung nach Anspruch 1, **dadurch gekennzeichnet, dass** die Öffnung eine Größe derart aufweist, dass immer nur ein Schüttgutpartikel (303) nach dem anderen durch die Öffnung hindurchfällt.

3. Vorrichtung nach Anspruch 2, **dadurch gekennzeichnet, dass** die Öffnung an der Position der verlängerten Ringachse des ringförmigen Elektronenstrahlerzeugers (301) angeordnet ist.

4. Vorrichtung nach Anspruch 2, **dadurch gekennzeichnet, dass** die untere Bodenwandung (404) mehrere Öffnungen (405) aufweist, die konzentrisch um die verlängerte Ringachse des ringförmigen Elektronenstrahlerzeugers (401) herum angeordnet sind.

5. Vorrichtung nach Anspruch 1, **dadurch gekennzeichnet, dass** die Öffnung (505) ringförmig um die verlängerte Ringachse des ringförmigen Elektronenstrahlerzeugers (501) herum ausgebildet ist.

6. Vorrichtung nach Anspruch 5, **dadurch gekennzeichnet, dass** die Öffnung (505) eine Ringbreite derart aufweist, dass bezüglich der Ringbreite lediglich ein Schüttgutpartikel hindurchpasst.

7. Vorrichtung nach Anspruch 1, **dadurch gekennzeichnet, dass** die Bodenwandung (504) ein kegelförmiges Element umfasst, dessen Kegelspitze an der Position der verlängerten Ringachse des ringförmigen Elektronenstrahlerzeugers (501) angeordnet ist.

8. Vorrichtung nach Anspruch 1, **gekennzeichnet durch** mindestens einen Schwingungserzeuger, der einen mechanischen Kontakt mit mindestens einem Bauteil der Einrichtung zum Vereinzeln von Schüttgutpartikeln aufweist.

9. Vorrichtung nach Anspruch 1, **dadurch gekennzeichnet, dass** mindestens ein Bauteil der Einrichtung zum Vereinzeln der Schüttgutpartikel höhenverstellbar ausgebildet ist.

10. Vorrichtung nach Anspruch 1, **gekennzeichnet durch** mindestens einen zylinderförmigen Elektronenreflektor (506), der sich durch die Ringöffnung des ringförmigen Elektronenstrahlerzeugers (501) erstreckt.

## Claims

1. Apparatus, comprising at least one electron beam generator (301) for generating accelerated electrons, to which bulk material particles (303) can be impinged during free fall along a perpendicular line
**characterized in that**
a) the electron beam generator (301) is of annular design, in which the electrons emitted from an annular cathode, accelerated in the direction of the ring axis and enter through an electron exit window;
b) the annular electron beam generator (301) is arranged in such a way that its ring axis is aligned perpendicularly or deviating by an angle of up to 45° from the vertical;
c) above the annular electron beam generator there is arranged a device belonging thereto for separating bulk material particles, the bottom wall (304) of which has at least one opening, from which bulk material particles (303) can fall out and from there through the annular aperture formed by the electron beam generator (301).

2. Apparatus according to Claim 1, **characterized in that** the opening has a size such that only one bulk material particle (303) after the other ever falls through the opening.

3. Apparatus according to Claim 2, **characterized in that** the opening is arranged at the position of the extended ring axis of the annular electron beam generator (301).

4. Apparatus according to Claim 2, **characterized in that** the lower bottom wall (404) has multiple openings (405), which are arranged concentrically around the extended ring axis of the annular electron beam generator (401).

5. Apparatus according to Claim 1, **characterized in that** the opening (505) is formed annularly about the extended ring axis of the annular electron beam generator (501).

6. Apparatus according to Claim 5, **characterized in that** the opening (505) has a ring width such that, with respect to the ring width, only one bulk material particle fits through.

7. Apparatus according to Claim 1, **characterized in that** the bottom wall (504) comprises a conical element, the conical tip of which is arranged at the position of the extended ring axis of the annular electron beam generator (501).

8. Apparatus according to Claim 1, **characterized by** at least one vibration generator, which has mechanical contact with at least one component of the device for separating bulk material particles.

9. Apparatus according to Claim 1, **characterized in that** at least one component of the device for separating the bulk material particles is designed to be vertically adjustable.

10. Apparatus according to Claim 1, **characterized by** at least one cylindrical electron reflector (506) which extends through the ring opening of the annular electron beam generator (501).

## Revendications

1. Arrangement, comprenant au moins un générateur de faisceau d'électrons (301) destiné à générer des électrons accélérés avec lesquels peuvent être bombardées des particules de matière en vrac (303) pendant la chute libre le long d'une verticale, **caractérisé en ce que**
a) le générateur de faisceau d'électrons (301) est réalisé en forme d'anneau, avec lequel les électrons émis et accélérés par une cathode en forme d'anneau sortent d'une fenêtre de sortie d'électrons en direction de l'axe de l'anneau ;
b) le générateur de faisceau d'électrons (301) en forme d'anneau est disposé de telle sorte que son axe d'anneau est orienté perpendiculairement ou avec un angle maximal de 45° par rapport à la verticale ;
c) un dispositif servant à la séparation des particules de matière en vrac associé au générateur de faisceau d'électrons en forme d'anneau est disposé au-dessus de celui-ci, sa paroi de fond (304) présentant au moins une ouverture hors de laquelle peuvent chuter des particules de matière en vrac (303) et, de là, chuter à travers l'anneau formé par le générateur de faisceau d'électrons (301) .

2. Arrangement selon la revendication 1, **caractérisé en ce que** l'ouverture présente une taille telle qu'il n'y a toujours qu'une seule particule de matière en vrac (303) après l'autre qui chute à travers l'ouverture.

3. Arrangement selon la revendication 2, **caractérisé en ce que** l'ouverture est disposée au niveau de l'axe d'anneau prolongé du générateur de faisceau d'électrons (301) en forme d'anneau.

4. Arrangement selon la revendication 2, **caractérisé en ce que** la paroi de fond inférieure (404) possède plusieurs ouvertures (405) qui sont disposées de manière concentrique autour de l'axe d'anneau prolongé du générateur de faisceau d'électrons (401) en forme d'anneau.

5. Arrangement selon la revendication 1, **caractérisé en ce que** l'ouverture (505) est réalisée en forme d'anneau autour de l'axe d'anneau prolongé du générateur de faisceau d'électrons (501) en forme d'anneau.

6. Arrangement selon la revendication 1, **caractérisé en ce que** l'ouverture (505) présente une largeur d'anneau telle que seule une particule de matière en vrac peut passer à travers en référence à la largeur d'anneau.

7. Arrangement selon la revendication 1, **caractérisé en ce que** la paroi de fond (504) comporte un élément de forme conique dont la pointe de cône est disposée au niveau de la position de l'axe d'anneau prolongé du générateur de faisceau d'électrons (501) en forme d'anneau.

8. Arrangement selon la revendication 1, **caractérisé par** au moins un générateur d'oscillations qui présente un contact mécanique avec au moins un composant du dispositif servant à la séparation de particules de matière en vrac.

9. Arrangement selon la revendication 1, **caractérisé en ce qu'**au moins un composant du dispositif servant à la séparation des particules de matière en vrac est configuré pour pouvoir être positionné en hauteur.

10. Arrangement selon la revendication 1, **caractérisé par** au moins un réflecteur d'électrons (506) de forme cylindrique qui s'étend à travers l'ouverture annulaire du générateur de faisceau d'électrons (501) en forme d'anneau.
